# EUROPEAN PATENT SPECIFICATION

(11) **EP 1 315 482 B1**
(45) Date of publication and mention of the grant of the patent: **22.11.2006**
(21) Application number: 01982227.9
(22) Date of filing: 30.08.2001
(51) Int. Cl.: A61K 9/62, A61K 31/575, A61P 3/06

(54) **WATER-DISPERSIBLE ENCAPSULATED STEROLS**
IN WASSER DISPERGIERBARE VERKAPSELTE STEROLE
STEROLS ENCAPSULES DISPERSABLES DANS L'EAU

(30) Priority: 01.09.2000 GB 0021498
(43) Date of publication of application: 04.06.2003
(60) Divisional application: 06122379.8
(73) Proprietor: Forbes Medi-Tech Inc., Vancouver, British Columbia V7Y 1K2 (CA)
(72) Inventor: AURIOU, Nicolas, CH-3012 Bern (CH)
(74) Representative: Jones, Helen M.M.
(86) International application number: PCT/EP2001/010044
(87) International publication number: WO 2002/017892

(56) References cited:
- EP-A- 0 289 636
- EP-A- 0 947 197
- WO-A-99/18977
- US-A- 3 881 005
- DATABASE BIOSIS [Online] BIOSCIENCES INFORMATION SERVICE, PHILADELPHIA, PA, US; October 1999 (1999-10) VISWANATHAN ARVIND: "Effect of degree of substitution of octenyl succinate starch on the emulsification activity on different oil phases." Database accession no. PREV200000323892 XP002190198 & JOURNAL OF ENVIRONMENTAL POLYMER DEGRADATION, vol. 7, no. 4, October 1999 (1999-10), pages 191-196, ISSN: 1064-7546

## Description

The invention relates to a method of manufacturing encapsulated forms of sterols (such as phytosterols, stanols, and esters or other derivatives thereof) which are water-dispersible, and to the encapsulated sterol powders themselves. The invention also concerns the use of such encapsulated phytosterols in the preparation of pharmaceutical or nutritional products, including food products and beverages, and the medical applications of such products.

### Background of the Invention

The benefit to cardiovascular health of consuming sterols from plant sources has been recognized for many years. Oils and fats are capable to a limited extent of dissolving free phytosterols, but the highly hydrophobic nature of phytosterols renders them insoluble and barely dispersible in aqueous media. Therefore, in order to facilitate dissolution of phytosterols in the micellar phase in the digestive tract, and thereby displace cholesterol from the micelles, it is usual to formulate phytosterols in fat- or oil-based food products. For solubility reasons such products are also easier to manufacture than non-oleaginous delivery vehicles. However, this places people suffering from hypercholesterolemia in a dilemma: medical advice dictates that they limit dietary fat intake, yet in order to benefit from the cholesterol-lowering effects of plant-derived sterols they must consume these in the form of fat-rich functional food.

Clearly, the consumer would prefer to ingest phytosterols in the form of low fat, or even fat-free foods, and in response the food processing industry has been concentrating its efforts on developing ways of providing sterols in alternative, non-fat based formats.

Means of dispersing sterol-like molecules in aqueous solutions are known in the art; for example, emulsification to create micelles of sterols in an aqueous medium can be achieved using standard food-grade surfactants or emulsifiers. However, the emulsions are frequently unstable, and the end-product tends to be poorly dispersed and leaves a waxy sensation in the mouth.

US-A-3,881,005, example 1, discloses a sitosterol powder which is produced by the steps of dissolving sorbitan mono stearate in water, adding starch hydrolysate and starch, adding sitosterol, homogenising, deaerating, pasteurising and spray drying the mixture.

EP-A-0947197 discloses a phytosterol powder which is produced by the steps of mixing a surfactant, multodextrin, starch and phytosterol in water and spray drying the mixture.

Database Biosis [on line] Biosciences Information Service, Philadelphia, PA, US, October 1999 (10) Viswanathan, Arvind, describes the effect of the degree of substitution of octenyl succinate starch on its emulsification activity.

WO-A-9918977 describes the use of macromolecules to solubilise steryl glycosides. The macromolecules include starch and modified starch as well as other components. The example utilises a single component macromolecule which is starch.

EP-A-0289636 uses surfactants to emulsify sterols into aqueous compositions.

The present invention concerns a method of optimizing phytosterol dispersion in an aqueous medium which overcomes the drawbacks of prior art techniques, and involves emulsification of the phytosterol solution with a surfactant to create micelles or vesicles, which are encapsulated with a mixture of starch and modified starch.

### Summary of the Invention

According to one aspect of the invention there is provided a method of preparing a water-dispersible powder containing encapsulated phytosterol, which comprises the steps of:
(a) in an aqueous medium, mixing the phytosterol with starch, modified starch and surfactant; and
(b) drying the product of step (a) to obtain a water-dispersible powder in which the phytosterol is encapsulated, wherein the modified starch is a starch alkenyl succinate.

According to a second aspect of the invention there is provided a composition comprising a phytosterol, starch and modified starch, wherein the modified starch is a starch alkenyl succinate and the phytosterol is encapsulated.

According to a third aspect of the invention there is provided a food or beverage product comprising the novel composition, and optionally further comprising one or more active ingredients selected from among: omega-3, omega-6 and omega-9 fatty acids, polyphenols, lipid-soluble antioxidants (e.g. tocopherol, tocotrienols, lycopene), water-soluble antioxidants (e.g. ascorbate), amino acids (e.g. arginine), dietary fibers, vitamins, and minerals.

According to another aspect of the invention there is provided a composition comprising a phytosterol, starch and modified starch, for use as a medicament wherein the modified starch is a starch alkenyl succinate and the phytosterol is encapsulated. In a further aspect of the invention there is provided use of a water-dispersible composition comprising a phytosterol, starch and modified starch, in the preparation of a medicament or nutritional formulation for the treatment or prevention of any of: elevated blood cholesterol levels, hypertriglyceridemia, coronary heart disease, diabetes, atherosclerosis, inflammation, osteoarthritis, breast cancer, colon cancer, and benign prostatic hyperplasia wherein the modified starch is starch alkenylsuccinate and the phytosterol is encapsulated.

### Detailed Description of the Invention

Emulsifiers such as sucrose fatty acid esters are known in their own right to be capable of emulsifying sterols, and have been used in lipid vesicles encapsulating phytosterols (e.g. US 5,405,615). Starch alkenyl esters, which are a form of modified starch, are known as emulsifier stabilizers and are also used in the food Industry to encapsulate oils, fats and flavours. However, a surprising and marked improvement in dispersibility and reduction in waxy feel was observed when these components were used together to create an aqueous dispersion of a phytosterol. As these components have not previously been used in combination the degree of improvement observed in terms of dispersion and organoleptic properties could not have been predicted. Furthermore, it is striking that the emulsifying/encapsulating method of the invention allows phytosterols to be concentrated into powder form, thereby opening up new possibilities for developing low-fat or non-fat food and drink products containing larger amounts of phytosterol than was previously feasible.

In the present context the generic term "sterol" or "phytosterol" is intended to encompass any member of the family of phytosterols, phytostanols, and esters or other derivatives thereof, and any mixture or combination thereof. Phytostanols are the 5 alpha saturated derivatives of phytosterols, which can be synthesized from phytosterols by hydrogenation. Phytosterols can be esterified with fatty acids, with glycosides, with acyl amino acids and with amino acids. In fact, we observe that esterification of phytosterols is superfluous in the context of the present invention, since the encapsulated phytosterols described herein are biologically active and easily incorporated into foodstuffs.

The phytosterols may be chemically synthesized, or may be derived from natural sources, including plants sources such as avocado, soy, rice bran, tall oil pitch or soap, shea nut, coconut, and plant oils, for example rapeseed, soya, maize, sunflower and sesame oils, as well as fish oils and other marine-animal oils. Some germ oils are very rich in phytosterols, wheat germ and oats being good examples. Non-exhaustive examples of plant sterols include sitosterol, stigmasterol, campesterol, brassicasterol, desmosterol, chalinosterol, poriferasterol, avenasterol, and clionasterol, and their corresponding stanols.

Representative examples of mixed sterols are those having the following compositions: 90-100% phytosterol + 0-10% phytostanol; 60-75% phytosterol + 25-40% phytostanol; 10-20% phytosterol + 80-90% phytostanol. Preferred phytosterol product types A, B, and C have the following composition (in % by weight of total sterol concentration):

| | A | B | C |
|---|---|---|---|
| | (e.g. Becel^{®}) | (e.g. Phytrol^{®}) | (e.g. Benecol^{®}) |
| Phytosterol type: | | | |
| Sitosterol | 30-65% | 45-60% | 2-5% |
| Campesterol | 10-40% | 10-14% | 1-5% |
| Stigmasterol | 6-30% | --- | --- |
| Brassicasterol | 0-9% | --- | --- |
| Sitostanol (Stigmastanol) | 0-10% | 18-30% | 60-70% |
| Campestanol | 0-6% | 2-6% | 20-30% |
| Minor Sterol | 6-10% | 6-10% | 4-8% |

The powdered composition of the invention may contain from about 25 to about 95% by weight of phytosterols, preferably about 60 to 90%, and most preferably about 70 to 80% by weight. In order to facilitate exposure to bile salts, phytosterols are preferably administered in very finely divided form. The smaller the particle size the better the dispersion obtained, and the less "sandy" the feeling in the mouth. A low mean particle size (e.g. between 10 and 100 microns, preferably lower than 50 microns) can be achieved by milling and sieving, or by following the "impact forces" technique described in WO 00/45648.

In one embodiment of the invention the only physiologically active component of the water-dispersible powder is the sterol component. In another embodiment the phytosterol micelles may also contain significant quantities of other bioactives, especially hydrophobic molecules which may be of benefit in preventing or treating cardiovascular disease or other medical conditions. Suitable candidate molecules include PUFAs, carotenoids (e.g. lycopene), tocopherols and tocotrienols.

The surfactant (emulsifier) used in the invention can be any type of ionic or non-ionic food-grade emulsifier, or mixtures thereof. In the context of the present invention it is generally preferred that the Hydrophilicity-Lipophilicity Balance (HLB) be high (more hydrophilic), i.e. between 8 and 18, preferably 10 to 17, and most preferably about 15 to 16, to ensure good micelle formation. Non-ionic emulsifiers such as sucrose fatty acid esters (sugar esters or sucro-esters, E473) comprising mono-, di- or tri-esters are one group of surfactants which satisfy this criterion. The HLB of sucrose fatty acid esters correlates with the content of monoester and can range up to about 18. Sucrose fatty acid esters are synthesized by esterification of fatty acids from natural triglycerides and sucrose. As sucrose has 8 hydroxyl groups, compounds ranging from mono- to octa-esters can be produced. Generally, sucrose fatty acid esters with more hydroxyl groups and fewer fatty acids (e.g. monoesters) are more hydrophilic (high HLB). Sucrose fatty acids having a monoester content of about 45 to 80% by weight of the total sugar ester are preferred.

Although fatty acids in the C₈ to C₂₄ range are suitable for esterifying sucrose, long chain saturated or unsaturated fatty acids of C₁₄ or longer are preferred. Long chain fatty acids include lauric, myristic, palmitic, stearic, oleic, behenic, erucic, elaidic, arachidic, arachidonic, linoleic, and linolenic acids. Laurate and stearate esters are preferred. Commercially available sugar esters include mixtures of sugar esters with different degrees of esterification. For example, mixtures of palmitic and stearic acids have been approved for food use in the EU and US. In some countries the total content of mono-, di-, and triesters must be a minimum of 80% to conform with food regulations.

Sucrose esters are readily hydrolyzed by digestive enzymes and metabolized by the body. Without wishing to be bound by theory, the hydrolysis of the sucrose esters may liberate phytosterol molecules from the encapsulated micelles *in situ* in the intestine, where they are concentrated and effective for displacement of cholesterol from bile micelles.

The sucrose fatty acid ester is preferably used in an amount of from about 0.01 to 10% by weight of the total powder composition, preferably in an amount of 0.1 % to 5%, and most preferably in an amount of 1 to 4%, especially about 2% by weight.

Although in a preferred embodiment of the invention sucrose fatty acid esters are the only emulsifiers used in the preparation of the water-dispersible phytosterol powder, it is also possible to use supplemental emulsifiers. Additional emulsifiers which can be employed in the invention include: anionic surfactants such as alcohol ether sulfates, alkyl sulfates, soaps and sulfosuccinates, sodium dodecyl sulfate (SDS), mono and diacylglycerides and derivatives thereof, e.g. Acetem^{®}, Lactem^{®}, Citrem^{®}, Datem^{®}; cationic surfactants such as quaternary ammonium compounds; zwitterionic surfactants such as alkyl betaine derivatives; amphoteric surfactants such as fatty amine sulfates, difatty alkyl triethanolamine derivatives, phospholipids, lecithins, lysolecithins; and non-ionic surfactants such as the polyglycol ether derivatives of aliphatic or cycloaliphatic alcohols, saturated fatty acids and alkylphenols, water-soluble polyethyleneoxy adducts onto polypropylene glycol and alkyl polypropylene glycol, nonylphenol polyethoxyethanols, castor oil polyglycol ethers, polypropylene/polyethylene oxide adducts, tributylphenoxy-polyethoxyethanol, polyethylene glycol, octylphenoxy-polyethoxyethanol, lanolin alcohols, polyethoxylated (POE) alkyl phenols, POE fatty amides, POE fatty alcohol ethers, POE fatty amines, POE fatty esters, poloxamers (7-19), POE glycol monoethers (13-16), polysorbates and sorbitan esters, and glyceroglycolipids.

As emulsion stabilizers, it is possible to use any products used for this purpose in food products. Appropriate emulsion stabilizers include, but are not limited to, lyophilic colloids such as polysaccharides (e.g. acacia, agar, alginic acid, carrageenan, guar gum, karaya gum, tragacanth, locust bean gum, xanthan gum, cellulose gums, modified food starches, cellulose and its methylated derivatives, microcrystalline cellulose), amphoterics (e.g. gelatin) and synthetic or semi-synthetic polymers (e.g. carbomer resins, cellulose ethers, carboxymethyl cellulose, carboxymethyl chitin, polyethylene glycol-n (ethylene oxide polymer H(OCH₂CH₂)ₙOH); finely divided solids including clays (e.g. attapulgite, bentonite, hectorite, kaolin, magnesium aluminium silicate and montmorillonite), microcrystalline cellulose oxides and hydroxides (e.g. aluminium hydroxide, magnesium hydroxide and silica); and cybotactic promoters/gellants Including amino acids, peptides, proteins, lecithin and other phospholipids and poloxamers.

When the phytosterol and surfactant, plus any additional emulsifiers, are thoroughly mixed at a high speed it is possible to create micelles having a diameter of from about 1 to about 400 microns, preferably from about 10 to 100 microns. The size of the particles can be measured using a Turbimeter, and quantified in terms of Nepthialic Turbidity Units (NTU). Preferred turbidity levels are greater than 2000 NTU. Alternatively, particle size can be assessed by means of laser light scattering, or using a spectrophotometer.

Starch is known as an excipient for dispersible powders of sitosterols (US 3,881,005), and is a component of the composition of the present invention. The content of starch in the dried powder product is in the range of 1 to 50% by weight, preferably 2 to 20%, and more preferably about 5 to 10% by weight. Optional excipients can be selected from those conventionally used in the preparation of pharmaceutical compositions, especially starch hydrolysate, cellulose, microcrystalline cellulose, dextrose, fructose, lactose, maltose, saccharose, mannitol, sorbitol, sucrose, com syrup solids, nonfat dry milk solids, casein, sodium caseinate, stearic acid, sodium stearate, magnesium stearate, fumed silicon dioxide, and vegetable oils. The fluidity of the powder can be improved through the use of flow conditioners or anti-caking agents, for instance tricalcium phosphate, talc, stearic acid and its salts, polyethylene glycol or fumed silicon dioxide.

In the context of the present invention modified starches are starch alkenyl succinates, particularly where the alkenyl group has 3 to 12 carbon atoms. Starch alkenyl succinate is obtainable by treating starch with alkenyl succinic anhydride under controlled pH conditions. The most preferred form of alkenyl ester starch is octenyl-succinate starch (OSA), also known as E1450, and approved for use in food in its sodium salt form. This compound is made by reacting starch with n-octenyl succinic anhydride at pH 8-8.5. The starch may be derived from any vegetable source including com, wheat, potato, tapioca, rice, sago and grain sorghum. Crosslinked starches can be obtained by chemical treatment or by extraction from naturally crosslinked plant starch sources, such as waxy corn. OSA is preferably obtained from waxy corn starch.

The modified starch preferably constitutes up to 30% by weight, preferably 1 to 25%, more preferably 2 to 10% by weight of the emulsion prior to spray-drying, and up to 60% by weight, preferably 5 to 40%, more preferably 10 to 25% by weight of the final spray-dried powder product.

If desired, one or more chelating agents such as citric acid, EDTA, phenylalanine, phosphoric acid, tartaric acid and tryptophan can be included in the encapsulated phytosterol product. Anti-oxidants which are suitable for inclusion in the product of the invention include water-soluble and lipid-soluble agents selected from ascorbic acid, sodium bisulfite, sodium sulfite, alkyl gallates, ascorbyl palmitate, t-butyl hydroquinone, butylated hydroxyanisole, butylated hydroxytoluene, hydroquinone, nordihydroguaiaretic acid, alpha-tocopherol and tocotrienols. Preservatives which may be used in the method of the invention are optionally selected from among the parabens (ethyl-, methyl- or propylparaben), sorbic acid, thiomersal, quaternary ammonium salts, benzyl alcohol, benzoic acid, chlorhexidine and phenylethanol. Further optional additives are osmotic agents (monovalent, divalent and trivalent electrolytes, monosaccharides and disaccharides); buffers or agents for pH adjustment (e.g. citric acid, tartaric acid, phosphoric acid, acetic acid, hydrochloric acid, salts of any of these acids, sodium hydroxide and sodium bicarbonate); colours and flavours.

One suitable method for preparation of water-dispersible phytosterol products is carried out in several stages. In a first step, the surfactant is added to a first volume of hot water (60 to 65°C), and after dissolution the mixture is cooled to approximately 30°C. Then the phytosterol is added and the mixture is agitated to ensure creation of an emulsion of micelles containing phytosterol. The emulsion can be prepared by shear mixing, vortexing, sonicating, microfluidizing or use of a French press. Then the starch and modified starch are blended with the micellar solution. The starch and modified starch may be added as a pre-mix or can be mixed in simultaneously or separately with the surfactant solution. The mixture is agitated vigorously to promote dispersion, and the mixing is continued until there are no more aggregations or clumps. Optionally, the order of addition of phytosterol and starch/modified starch to the surfactant can be reversed.

In the second stage the aqueous mix is either spray-dried (inlet temperature about 160°C, outlet temperature about 80°C) or freeze-dried, or dried by rotary evaporation or vacuum drying to create a powder of starch/modified starch-encapsulated phytosterol. Spray-drying is the preferred process for removal of water from the sterol composition. During spray drying the emulsion is broken up into droplets of a desired size by means of a spray nozzle, spinning disc, or apertured centrifugal atomizer. Then moisture is removed in a drying environment to solidify the coating material around the droplets to form solid particles (powder). The powder product produced by atomization in accordance with the invention has a particle size distribution which promotes dispersion in aqueous liquids. The moisture content of the finished powder product should be low, i.e. about 3% or less, preferably between about 0.5% and 1 % by weight, in order to ensure a good powder fluidity.

Homogenization (in two steps, at 250 and 50 bar), deaeration and sterilization (e.g. pasteurization) can be carried out on the aqueous mixture after all of the components have been thoroughly mixed, before the drying step. Homogenization is preferably carried out under a pressure of about 5000 psig (35 MPa gauge). Deaeration or degassing is achieved by allowing the mixture to stand, or by mechanical intervention. A preferred procedure is to slowly flow the dispersion in a thin film over a plate inside of a container on which vacuum has been drawn to a pressure of 30 inches (763mm) of mercury or more. The deaerated dispersion is subjected to a pasteurization operation at a temperature of from 63°C to 69°C for a period of approximately 6 hours or more. Optionally, the spray-dried or freeze-dried product is sterilized by heating or other conventional means.

The water-dispersibility properties of the inventive powder composition are evidenced by the ease and rapidity of dispersion, the lack of sedimentation and floating, and the low opacity (milkiness) of the resulting solution, as can be judged by the naked eye. Alternatively the method for aqueous dispersibility testing disclosed in WO 99/63841 can be adopted. The hydrated product also has excellent palatability and mouth-feel (lack of waxy sensation), which is easily discernible in taste comparisons with mixtures of non-encapsulated phytosterols with water, and with allegedly water-dispersible phytosterol powders obtainable by conventional techniques.

This invention makes possible the provision of phytosterols in oral pharmaceutical dosage forms, particularly solid forms, including tablets, capsules, dragées, granules, pellets, chewable dosage forms, solutions, syrups, lozenges, and powders. Customary pharmaceutical excipients, diluents and stabilizers may be employed.

Pharmaceutical binders useful for making tablets or dragée cores incorporating the encapsulated powder product of the invention include starch pastes (from corn, wheat, rice or potato starch), gelatin, tragacanth, methylcellulose and polyvinylpyrrolidone (PVP). Disintegrators may also be incorporated into solid pharmaceutical formulations, examples being starches, carboxymethyl starch, cross-linked PVP, agar, alginic acid and its salts. Dragée cores are provided with suitable, optionally enteric, coatings, there being used *inter alia* concentrated sugar solutions which may contain gum arabic, talc, PVP, polyethylene glycol and/or titanium dioxide, or coating solutions in suitable organic solvents or solvent mixtures or, for the preparation of enteric coatings, solutions of suitable cellulose preparations, such as acetylcellulose phthalate or hydroxypropylmethylcellulose phthalate.

Other orally administrable pharmaceutical compositions comprising the product of the invention are hard gelatin capsules and also soft, sealed capsules consisting of gelatin and a plasticiser, such as glycerol or sorbitol. The hard gelatin capsules may comprise the active ingredient in the form of granules, for example in admixture with fillers, such as lactose, dextrose, mannitol, binders such as starches and/or glidants, such as talc, colloidal silica or magnesium stearate and, if desired, stabilisers. In soft capsules the active ingredient is preferably dissolved or suspended in suitable liquids, such as fatty oils, paraffin oil or liquid polyethylene glycols, additional stabilisers being optional.

The components of the powder are non-toxic and safe for use in food. The spray-dried phytosterol-containing product can be included in any hydrophilic or water-based food or beverage formulation, especially low fat or fat-free products. By "low fat product" is meant a product in which fewer than 5%, preferably fewer than 2%, of the total calories are contributed by lipids. By "fat-free product" is meant a product in which lipids are practically undetectable, i.e. contributing less than 1%, or even less than 0.5% of the total calories.

The nutritional breakdown of the food and drink products can vary according to the nature of the product. In general, carbohydrate and/or protein contribute the bulk of the calorific content of the product.

For example, it may be desired to include dispersed phytosterol in soft drinks (especially low fat or low calorie drinks) such as milk, juices, flavoured/unflavoured mineral water, carbonated drinks, sports drinks, soy-based beverages, meal replacement drinks, yoghurt drinks, and smoothies. Conveniently, the encapsulated phytosterol powder of the invention has less of a tendency to foam when agitated in a beverage than do known sterol powders, which allows drinkable formulations to be dispensed in diverse forms of packaging.

As the encapsulated sterol is provided in powder form it is ideally suited to being introduced into solid food products (powders, tablets, bars, and the like), as well as liquid comestibles. Sterols in liquid emulsions are less flexible in this regard. Suggested food products include low-fat or fat-free comestibles of any sort, especially starchy products like bread, crackers, biscuits, toast, breakfast cereals, cereal bars, muesli bars, processed rice products, pasta, other baked goods, confectioneries, chocolate, and the like. Fermented solid or liquid products may also incorporate encapsulated phytosterols. It is preferred that in the finished food or drink product the phytosterol content does not exceed 20% by weight of the product, and is preferably 0.1 to 8% by weight.

The pharmaceutical, food or drink products may be supplemented with other health-promoting ingredients, particularly ingredients known to have benefits for the cardiovascular system. Non-limiting examples are omega-3, omega-6 or omega-9 fatty acids, polyphenols, lipid-soluble antioxidants (e.g. tocopherol, tocotrienols, lycopene), water-soluble antioxidants (e.g. ascorbate), amino acids (e.g. arginine), dietary fibers, vitamins, minerals and the like.

Pharmaceutical, food or beverage products incorporating water-dispersible phytosterols can be safely consumed by anyone, but conveniently form part of the diet of those with a propensity to having high blood cholesterol levels. According to one aspect of the invention a method is provided for preventing or treating high blood cholesterol levels comprising administering, to a person in need of such treatment, a water-dispersible powder comprising one or more phytosterols in an amount effective to lower blood cholesterol, wherein the water-dispersible powder comprises phytosterol(s), starch and modified starch. By "high blood cholesterol" is meant: over 200mg/dl, especially over 240mg cholesterol/dl blood; and/or a ratio total cholesterol/HDL of 5:1 or greater; and/or an LDL blood concentration of greater 130mg/dl, especially over 160mg/dl. The pharmaceutical and nutritional products of the invention play a role in reducing blood cholesterol levels and thereby preventing cardiovascular disease and heart disease.

Aside from hypercholesterolemia, other indications for which phytosterols in encapsulated form may deliver a health benefit include: hypertriglyceridemia, coronary heart disease, diabetes, atherosclerosis, inflammation, osteoarthritis, breast cancer, colon cancer, and benign prostatic hyperplasia.

### EXAMPLES

### Example 1

An encapsulated phytosterol water-dispersible powder made in accordance with the invention has the following composition:

| **Ingredient** | **% by total weight** |
|---|---|
| Phytosterol* | 74.3 |
| OSA** | 15.8 |
| Com starch | 6.4 |
| sucro-ester*** | 2 |
| tricalcium phosphate | 1 |
| water | 0.5 |

| | |
|---|---|
| * The raw material phytosterol is a mixture of beta-sitosterol (58 wt %), sitostanol (20 wt %), campesterol (11 wt %) and campestanol (3 wt %), plus minor content of other phytosterols. **Sodium Octenyl succinate starch sold by Roquette, Lestrem, France ***S 1670: a Ryoto sucrose stearate ester available from Mitsubishi-Kagaku foods | |

### Example 2

### Detailed protocol for manufacture of encapsulated water-dispersible powder of Example 1:

70g of sugar ester S1670 (Ryoto) was added to 3 litres of water preheated to 60°C. The mixture was agitated until all of the sugar ester had gone into solution. The mixture was then cooled by addition of 3.5 litres of cold water (approx. 15°C). 560g of octenyl succinate of starch (OSA) was added to this surfactant-cold water combination. The resulting combination was then mixed vigorously until dissolution was complete. Then, 52.5g of tricalcium phosphate and 176g of corn starch were added together to the solution of surfactant and OSA. The resulting dispersion was agitated vigorously until all the ingredients were in suspension and uniformly blended.

2638g of finely-divided phytosterol was added to the blend, and vigorous agitation was resumed until the phytosterol was thoroughly dispersed and uniformly suspended.

The aqueous suspension was deaerated by flowing the dispersion in a thin film over a flat plate inside a vacuum chamber.

Then the deaerated suspension was homogenized using a Rannie homogenizer in a two-step process at 250/50 bar.

Pasteurization of the deaerated suspension was carried out by subjecting the phytosterol suspension to a temperature of 63 to 69°C for 6 hours.

Finally, the phytosterol suspension was spray-dried at a rate of 20 litres per hour. The inlet air temperature to the spray dryer was held at about 160°C and the accompanying outlet air temperature was about 80°C.

The water-dispersible powder prepared in this manner dispersed readily in all tested pharmaceutical and nutritional, solid and liquid formats.

### Example 3

### Protocols for testing powder of Example 1 (water-dispersibility, mouth-feel)

The hydrophilicity and dispersibility in aqueous media of the water-dispersible powders of the invention can be determined by simple laboratory tests.

**A.** For example, it is possible to measure the necessary time to full hydration of the powder by laying 0.5g of the powder on the surface of 50ml cold water (25°C) in a beaker. By visual inspection, hydration is judged to be complete when there is a colour change from white to greyish-white.

In the same experimental set-up, the sedimentation characteristics of the powder can be determined by measuring the time elapsed before it is possible to see a regular flow of powder particles towards the base of the beaker (in a depth of 2cm water, without agitation).

The encapsulation efficiency can be assessed by measuring the surface area of floating particles on the surface of the water. As hydrophobic particles, phytosterol particles need to be coated in hydrophilic and wetting compounds in order to allow sedimentation in an aqueous medium. Insufficiently-coated particles float on the surface.

(Note: non-encapsulated phytosterol does not hydrate, and floats on the surface of cold water even after mixing).

### Comparative Test Results:

| **Water property** | **Sample of Example 1** | **Sample of US 3,881,005**** |
|---|---|---|
| Hydration (sec) | 14 | 13 |
| Sedimentation (sec) | 62 | 50 |
| Floating (cm²) | <2 | ≥2 |

| | | |
|---|---|---|
| **This sample was produced in accordance with the method laid out in Example 1 of US 3,881,005, but using the phytosterol described in relation to Example 1 above. | | |

Clearly, the powder produced in accordance with the present invention has less of a tendency to float then does that of the prior art (US 3,881,005), indicating its superior hydration and aqueous dispersion properties.

**B.** The turbidity of the dispersions tested in part A was judged at 2 hour and 24 hour time points. 2ml of supernatant was removed from the beaker, and the percentage of light transmission (%T) at 400nm was measured using a spectrophotometer. When %T is 100 the solution is totally clear and transparent, whereas a value of 0 indicates opacity.

| **Transmission (%T)** | **Sample of Example 1** | **Sample of US 3,881,005**** |
|---|---|---|
| after 2h | 45.8 | 37.6 |
| after 24h | 69.1 | 65.1 |

It is evident that the powder of the present invention is dispersed more rapidly and more effectively (in the absence of agitation) than is the powder of Example 1 of US 3,881,005.

C. An additional consideration with regard to beverage formulation is the generation of foam as a result of vigorous mixing. The extent of foam formation can be evaluated by reading off the thickness of the foam layer created after mixing 1 g of powder in 100ml deionized water using magnetic agitation at 25°C.

| | **Sample of Example 1** | **Sample of US 3,881,005**** |
|---|---|---|
| Foam formation (mm) | 3 | 10 |
| Foam stability (min) | <1 | >1 |

It can be seen that the encapsulated powder of the claimed invention produces less foam, and shorter-lived foam, than does the powder of US 3,881,005, Example 1.

D. Dispersions in water of the powder of Example 1, and of that produced in accordance with the method of Example 1 of US 3,881,005, were compared by a panel of tasters. The tasters detected less of an unpleasant, waxy mouthfeel with the powder of the present invention, when compared to the prior art powder.

## Claims

1. A method for preparing a water-dispersible powder comprising encapsulated phytosterol, which comprises the steps of:
(a) in an aqueous medium, mixing the phytosterol with starch, modified starch and surfactant; and
(b) drying the product of step (a) to obtain a water-dispersible powder in which the phytosterol is encapsulated, wherein the modified starch is a starch alkenyl succinate.

2. A method according to claim 1 wherein the modified starch is starch octenyl succinate.

3. A method according to claim 1 or claim 2 wherein said surfactant has a HLB value of between 8 and 18.

4. A method according to any preceding claim wherein said surfactant comprises a sucrose fatty acid ester.

5. A method according to any preceding claim wherein the mixing in step (a) is achieved by vigorous agitation.

6. A method according to any preceding claim which comprises the steps of dissolving the surfactant in the aqueous medium to form a solution, followed by the step of adding the starch and modified starch to said solution to form a mixture, followed by addition of the phytosterol to the mixture.

7. A method according to any preceding claim wherein step (b) is a spray-drying step.

8. A method according to any preceding claim which comprises a series of intervening steps between steps (a) and (b), said intervening steps being the steps of homogenization, deaeration, and pasteurization.

9. A composition comprising a phytosterol, starch and modified starch, wherein the modified starch is a starch alkenyl succinate and the phytosterol is encapsulated.

10. A composition according to claim 9 comprising micelles containing the phytosterol and a surfactant, said micelles being coated with the starch and modified starch.

11. A composition according to claim 9 or claim 10 wherein the modified starch is starch octenyl succinate.

12. A composition according to claim 10 or claim 11 wherein the surfactant has a Hydrophilicity-Lipophilicity Balance (HLB) in the range or 8 to 18.

13. A composition according to any of claims 10 to 12 wherein said surfactant is a sucrose fatty acid ester.

14. A composition according to claim 13 wherein said sucrose fatty acid ester is sucrose stearate.

15. A food or beverage product comprising the composition of any of claims 9 to 14.

16. A food or beverage product according to claim 15 which further comprises one or more active ingredients selected from among: omega-3, omega-6 and omega-9 fatty acids, polyphenols, lipid-soluble antioxidants (e.g. tocopherol, tocotrienols, lycopene), water-insoluble antioxidants (e.g. ascorbate), amino acids (e.g. arginine), dietary fibers, vitamins, and minerals.

17. A composition according to any of claims 9 to 14, for use as a medicament.

18. Use of a water-dispersible composition comprising a phytosterol, starch and modified starch, in the preparation of a medicament or nutritional formulation for the treatment or prevention of any of: high blood cholesterol levels, hypertriglyceridemia, coronary heart disease, diabetes, atherosclerosis, inflammation, osteoarthritis, breast cancer, colon cancer, and benign prostatic hyperplasia, wherein the modified starch is starch alkenyl succinate and the phytosterol is encapsulated.

## Patentansprüche

1. Verfahren zur Herstellung eines wasserdispergierbaren Pulvers, umfassend verkapseltes Phytosterol, welches umfasst die Schritte:
(a) Mischen des Phytosterols mit Stärke, modifizierter Stärke und Tensid in einem wässrigen Medium; und
(b) Trocknen des Produkts von Schritt (a), um ein wasserdispergierbares Pulver zu erhalten, in dem das Phytosterol verkapselt ist, wobei die modifizierte Stärke ein Stärke-Alkenylsuccinat ist.

2. Verfahren nach Anspruch 1, bei dem die modifizierte Stärke Stärke-Octenylsuccinat ist.

3. Verfahren nach Anspruch 1 oder Anspruch 2, bei dem das Tensid einen HLB-Wert zwischen 8 und 18 aufweist.

4. Verfahren nach irgendeinem vorhergehenden Anspruch, bei dem das Tensid einen Sucrosefettsäureester umfasst.

5. Verfahren nach irgendeinem vorhergehenden Anspruch, bei dem das Mischen in Schritt (a) durch heftige Bewegung erreicht wird.

6. Verfahren nach irgendeinem vorhergehenden Anspruch, welches umfasst die Schritte des Auflösens des Tensids in dem wässrigem Medium zur Bildung einer Lösung, gefolgt von dem Schritt der Zugabe der Stärke und der modifizierten Stärke zur Lösung zur Bildung einer Mischung, gefolgt von der Zugabe des Phytosterols zur Mischung.

7. Verfahren nach irgendeinem vorhergehenden Anspruch, bei dem Schritt (b) ein Sprühtrocknungsschritt ist.

8. Verfahren nach irgendeinem vorhergehenden Anspruch, welcher eine Reihe von dazwischenliegenden Schritten zwischen den Schritten (a) und (b) umfasst, wobei die dazwischenliegenden Schritte die Schritte der Homogenisierung, Entgasung und Pasteurisierung sind.

9. Zusammensetzung, umfassend ein Phytosterol, Stärke und modifizierte Stärke, wobei die modifizierte Stärke ein Stärke-Alkenylsuccinat ist und das Phytosterol verkapselt ist.

10. Zusammensetzung nach Anspruch 9, umfassend Mizellen, die das Phytosterol und ein Tensid enthalten, wobei die Mizellen mit der Stärke und der modifizierten Stärke überzogen sind.

11. Zusammensetzung nach Anspruch 9 oder Anspruch 10, bei dem die modifizierte Stärke Stärke-Octenylsuccinat ist.

12. Zusammensetzung nach Anspruch 10 oder Anspruch 11, bei dem das Tensid ein Hydrophilie-Lypophilie-Gleichgewicht (HLB) im Bereich von 8 bis 18 aufweist.

13. Zusammensetzung nach irgendeinem der Ansprüche 10 bis 12, bei dem das Tensid ein Sucrosefettsäureester ist.

14. Zusammensetzung nach Anspruch 13, bei dem der Sucrosefettsäureester Sucrosestearat ist.

15. Nahrungsmittel- oder Getränkeprodukt, umfassend die Zusammensetzung nach irgendeinem der Ansprüche 9 bis 14.

16. Nahrungsmittel- oder Getränkeprodukt nach Anspruch 15, welches ferner einen oder mehrere Wirkstoffe ausgewählt aus: Omega-3-, Omega-6- und Omega-9-Fettsäuren, Polyphenolen, lipidlöslichen Antioxidationsmitteln (z.B. Tocopherol, Tocotrienolen, Lycopin), wasserunlöslichen Antioxidationsmitteln (z.B. Ascorbat), Aminosäuren (z.B. Arginin), Nahrungsmittelfasern, Vitaminen und Mineralien umfasst.

17. Zusammensetzung nach irgendeinem der Ansprüche 9 bis 14 zur Verwendung als Medikament.

18. Verwendung einer wasserdispergierbaren Zusammensetzung, umfassend ein Phytosterol, Stärke und modifizierte Stärke, bei der Herstellung eines Medikaments oder einer Nahrungsmittelformulierung zur Behandlung oder Verhütung von hohen Blutcholesterinspiegeln, Hypertriglyceridämie, koronarer Herzerkrankung, Diabetes, Arteriosklerose, Entzündung, Osteoarthritis, Brustkrebs, Dickdarmkrebs und benigner Prostatahyperplasie, wobei die modifizierte Stärke Stärke-Alkenylsuccinat ist und das Phytosterol verkapselt ist.

## Revendications

1. Procédé pour préparer une poudre dispersible dans l'eau comprenant du phytostérol encapsulé, qui comprend les étapes de :
(a) dans un milieu aqueux, mélanger le phytostérol avec de l'amidon, de l'amidon modifié et un tensioactif ; et
(b) sécher le produit de l'étape (a) pour obtenir une poudre dispersible dans l'eau dans laquelle le phytostérol est encapsulé, où l'amidon modifié est un alcénylsuccinate d'amidon.

2. Procédé selon la revendication 1 où l'amidon modifié est l'octénylsuccinate d'amidon.

3. Procédé selon la revendication 1 ou la revendication 2 où ledit tensioactif a une valeur HLB entre 8 et 18.

4. Procédé selon l'une quelconque des revendications précédentes où ledit tensioactif comprend un ester d'acide gras et de saccharose.

5. Procédé selon l'une quelconque des revendications précédentes où le mélange dans l'étape (a) est réalisé par agitation vigoureuse.

6. Procédé selon l'une quelconque des revendications précédentes qui comprend les étapes de dissolution du tensioactif dans le milieu aqueux pour former une solution, suivie par l'étape d'addition de l'amidon et de l'amidon modifié à ladite solution pour former un mélange, suivie par l'addition du phytostérol au mélange.

7. Procédé selon l'une quelconque des revendications précédentes où l'étape (b) est une étape de séchage par dispersion.

8. Procédé selon l'une quelconque des revendications précédentes qui comprend une série d'étapes intermédiaires entre les étapes (a) et (b), lesdites étapes intermédiaires étant les étapes d'homogénéisation, de désaération et de pasteurisation.

9. Composition comprenant un phytostérol, de l'amidon et de l'amidon modifié, où l'amidon modifié est un alcénylsuccinate d'amidon et le phytostérol est encapsulé.

10. Composition selon la revendication 9 comprenant des micelles contenant le phytostérol et un tensioactif, lesdites micelles étant revêtues de l'amidon et de l'amidon modifié.

11. Composition selon la revendication 9 ou la revendication 10 où l'amidon modifié est l'octénylsuccinate d'amidon.

12. Composition selon la revendication 10 ou la revendication 11 où le tensioactif a une balance hydrophile-lipophile (HLB) dans la plage de 8 à 18.

13. Composition selon l'une quelconque des revendications 10 à 12 où ledit tensioactif est un ester d'acide gras et de saccharose.

14. Composition selon la revendication 13 où ledit ester d'acide gras et de saccharose est le stéarate de saccharose.

15. Produit de type aliment ou boisson comprenant la composition selon l'une quelconque des revendications 9 à 14.

16. Produit de type aliment ou boisson selon la revendication 15 qui comprend en outre un ou plusieurs ingrédients actifs choisis parmi : les acides gras oméga-3, oméga-6 et oméga-9, les polyphénols, les antioxydants solubles dans les lipides (par exemple tocophérol, tocotriénols, lycopène), les antioxydants insolubles dans l'eau (par exemple ascorbate), les aminoacides (par exemple arginine), les fibres alimentaires, les vitamines et les minéraux.

17. Composition selon l'une quelconque des revendications 9 à 14 destinée à être utilisée comme médicament.

18. Utilisation d'une composition dispersible dans l'eau comprenant un phytostérol, de l'amidon et de l'amidon modifié, dans la préparation d'un médicament ou d'une formulation nutritionnelle pour le traitement ou la prévention de l'un quelconque parmi : des niveaux élevés de cholestérol sanguin, l'hypertriglycéridémie, la maladie coronarienne, le diabète, l'athérosclérose, l'inflammation, l'arthrose, le cancer du sein, le cancer du côlon et l'adénome prostatique, où l'amidon modifié est un alcénylsuccinate d'amidon et le phytostérol est encapsulé.
